# EUROPEAN PATENT APPLICATION

(11) **EP 4 797 309 A1**
(43) Date of publication of application: **26.08.2026**
(21) Application number: 24885027.3
(22) Date of filing: 04.11.2024
(51) Int. Cl.: H01J 35/02, H01J 35/14, H01J 35/06, A61B 6/03, A61B 6/40

(54) **RAY SOURCE ASSEMBLY, RAY CONTROL METHOD, AND COMPUTER-READABLE STORAGE MEDIUM**

(30) Priority: 02.11.2023 CN 202311456512
(71) Applicant: Shanghai United Imaging Healthcare Co., Ltd., Shanghai 201807 (CN)
(72) Inventor: HE, Tao, Shanghai 201807 (CN); ZHOU, Jiawen, Shanghai 201807 (CN)
(74) Representative: Murgitroyd & Company
(86) International application number: PCT/CN2024/129664
(87) International publication number: WO 2025/093032

(57) **Abstract**

A ray source assembly, a ray control method, and a computer-readable storage medium. The ray source assembly comprises: an anode end (401), configured to receive an electron beam and generate rays on the basis of the received electron beam; a cathode end (402), configured to emit the electron beam to the anode end (401); and an electron beam control portion (403), disposed between the cathode end (402) and the anode end (401) and configured to adjust, by means of a control voltage, the number of electrons reaching the anode end (401) in the electron beam. The method can improve current control efficiency.

## Description

The present application claims the priority of the Chinese patent application No. 202311456512.4, titled "Ray Source Assembly, Ray Control Method and Computer-Readable Storage Medium" and filed on November 2, 2023, the entire content of which is incorporated into the present application by reference.

### TECHNICAL FIELD

The present application relates to the field of medical technology, particularly to a ray source assembly, a ray control method, and a computer-readable storage medium.

### BACKGROUND

Medical scanning devices including Computed Tomography (CT) devices need to perform dose modulation (DOM) during a scanning process. That is, the scanning dose of the medical scanning device can be adjusted according to requirements during the scanning process.

Taking a CT device as an example, since the scanning dose is positively correlated with tube current, the filament current of the tube in the CT device is currently adjusted to change the temperature of the filament current, thereby adjusting the number of electrons emitted by the filament, so as to control the tube current of the CT device.

However, the above-mentioned way of controlling the tube current is limited by the temperature change rate of the filament material, resulting in low current control efficiency. Therefore, how to improve the current control efficiency is a key research topic for those skilled in the art.

### SUMMARY OF THE INVENTION

In one aspect, the present application provides a ray source assembly, including:
an anode end configured to receive an electron beam and generate rays based on the received electron beam;
a cathode end configured to emit the electron beam to the anode end; and
an electron beam control unit disposed between the cathode end and the anode end, and configured to adjust the number of electrons in the electron beam that reach the anode end through a control voltage.

In some embodiments, the electron beam control unit includes at least two voltage control terminals, the at least two voltage control terminals form a potential difference with respect to the cathode end, and the direction of the potential difference is consistent with the movement direction of the electron beam.

In some embodiments, the electron beam control unit is configured such that the magnitude of the tube current formed by the electron beam is negatively correlated with the potential difference between the cathode end and the electron beam control unit.

In some embodiments, the control voltage is a negative voltage and lower than the voltage of the cathode end, and the electron beam control unit is configured such that the magnitude of the tube current formed by the electron beam is negatively correlated with the absolute value of the control voltage.

In some embodiments, the electron beam control unit is further configured to control the cross-sectional area of the electron beam and/or the position where the electron beam reaches the anode end through the control voltage.

In some embodiments, the electron beam control unit includes a first voltage control terminal and a second voltage control terminal respectively located on two opposite sides relative to the movement direction of the electron beam.

In some embodiments, the first voltage control terminal and the second voltage control terminal are configured to control the number of electrons passing by the first voltage control terminal and the second voltage control terminal along the movement direction of the electron beam based on corresponding control voltages.

In some embodiments, the first voltage control terminal and the second voltage control terminal are configured to control deflection of the electron beam based on the corresponding control voltages.

In some embodiments, the ray source assembly further includes:
an additional electron beam control unit configured to control the cross-sectional area of the electron beam and/or the position where the electron beam reaches the anode end by means of an additional control voltage.

In some embodiments, the additional control voltage is a negative voltage and lower than the voltage of the cathode end, and the additional electron beam control unit is configured such that the cross-sectional area of the electron beam is negatively correlated with the absolute value of the additional control voltage.

In some embodiments, the additional electron beam control unit includes a first additional voltage control terminal and a second additional voltage control terminal, the first additional voltage control terminal provides a first additional control voltage, the second additional voltage control terminal provides a second additional control voltage, and the additional electron beam control unit is configured to control deflection of the electron beam based on the first additional control voltage and the second additional control voltage.

In some embodiments, along the movement direction of the electron beam, the electron beam control unit is located upstream of the additional electron beam control unit.

In some embodiments, the ray source assembly further includes:
a magnetic control structure configured to control at least one of the cross-sectional area of the electron beam, the position where the electron beam reaches the anode end, and the number of electrons in the electron beam.

In another aspect, the present application provides a ray control method applied to the ray source assembly according to any one of the above embodiments, including:
determining a target tube current of a ray source according to a preset ray dose; and
determining the control voltage according to the target tube current.

In another aspect, the present application provides a computer-readable storage medium having a computer program stored therein. The computer program, when executed by a processor, implements the above-mentioned ray control method.

The details of various embodiments of the present invention will be set forth in the accompanying drawings and the description below. Based on the records in the specification, the accompanying drawings and the claims, those skilled in the art will easily understand other features, solved problems and technical effects of the present invention.

### BRIEF DESCRIPTION OF THE DRAWINGS

In order to explain the technical solutions in the embodiments of the present application or the related art more clearly, the accompanying drawings required for describing the embodiments or the related art will be briefly introduced below. Obviously, the accompanying drawings described below are only some embodiments of the present application, and for those of ordinary skill in the art, other accompanying drawings can be obtained according to these drawings without creative work.
FIG. 1 is a schematic diagram of dose modulation.
FIG. 2 is a schematic diagram of an elliptical model of a scanned object in the Z direction.
FIG. 3 is a schematic diagram of tube current adjustment limits.
FIG. 4 is a schematic structural diagram of a ray source assembly in embodiments of the present application.
FIG. 5 is a schematic structural diagram of another ray source assembly in embodiments of the present application.
FIG. 6 is a schematic structural diagram of another ray source assembly in embodiments of the present application.
FIG. 7 is a schematic structural diagram of another ray source assembly in embodiments of the present application.
FIG. 8 is a schematic structural diagram of another ray source assembly in embodiments of the present application.
FIG. 9 is a schematic diagram of a dose modulation in embodiments of the present application.
FIG. 10 is an application environment diagram of a ray control method in embodiments of the present application.
FIG. 11 is a schematic flow chart of a ray control method in embodiments of the present application.
FIG. 12 is a structural block diagram of a ray control and adjustment device in embodiments of the present application.
FIG. 13 is an internal structural diagram of a computer device in embodiments of the present application.

### DETAILED DESCRIPTION OF THE EMBODIMENTS

In order to make the objectives, technical solutions and advantages of the present application clear, the present application will be described in further detail below with reference to the accompanying drawings and embodiments. It should be understood that the specific embodiments described herein are only used to explain the present application and are not intended to limit the present application.

Dose modulation plays an important role in reducing the scanning dose and improving the signal-to-noise ratio of images, and can be applied to imaging devices such as CT (Computed Tomography), XR (X-ray imaging) and DR (Digital Radiography), or other radiotherapy devices. Taking the application of dose modulation to a CT device as an example, the dose modulation of the CT device mainly includes Z-direction dose modulation and X-direction dose modulation, where the Z direction is the axial direction of the scanning bed in the CT device, and the X direction is the direction of the optical plane in the CT device.

Taking the Z direction as an example, a scanned object on the scanning bed is approximated as an elliptical model, and ellipses with different axial lengths in the elliptical model can be determined in the Z direction. At positions with large attenuation, the axial length of the ellipse is also larger, and the required scanning dose is larger. Since the scanning dose is positively correlated with tube current, the tube current also needs to be increased at positions with large attenuation.

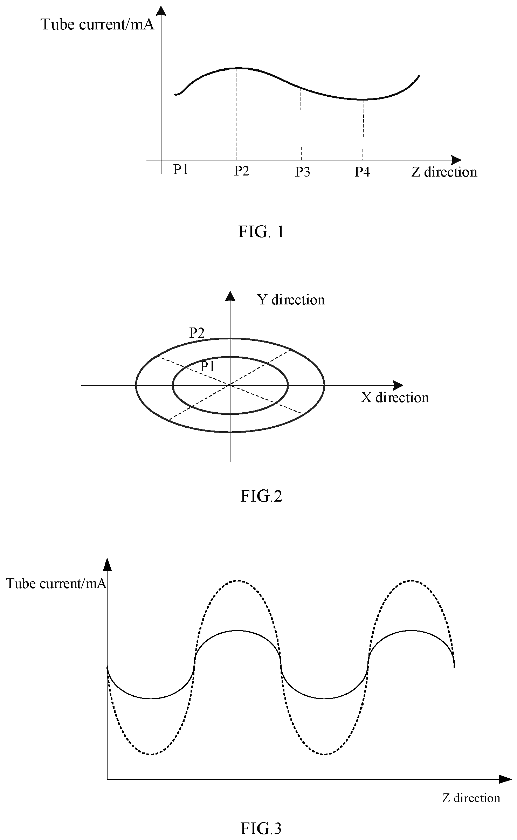

FIG. 1 is a schematic diagram of dose modulation, and FIG. 2 is a schematic diagram of an elliptical model of a scanned object in the Z direction. As shown in FIG. 1, P1 to P4 respectively represent different attenuation positions in the Z direction, and the required tube current varies at different attenuation positions. FIG. 2 shows ellipses formed at P2 and P1 with different axial lengths in the Z direction. With reference to FIG. 1, it can be seen that, for example, in the same direction in the X-Y plane, the axial length of the ellipse corresponding to P2 is larger than that corresponding to P1, and the tube current required at P2 is larger than that at P1.

At present, dose modulation is realized by adjusting the filament current of a tube in the CT device, but this method is limited by the temperature change rate of the filament material and the electron emission capacity, so it is difficult for the tube current of the CT device to change rapidly between different magnitudes.

FIG. 3 is a schematic diagram of tube current adjustment limits. As shown in FIG. 3, the dashed line is the ideal change curve of the tube current during the dose modulation process, and the solid line is the actual change curve of the tube current during the modulation process. It can be seen that due to the limitations of filament materials and other factors, the upper limit that the actual tube current can reach during the dose modulation process is lower than the upper limit of the theoretical tube current, and the lower limit that the actual tube current can reach is higher than the lower limit of the theoretical tube current. It can be seen that the way of adjusting the filament current in the actual modulation process has a large gap with the theoretical modulation curve, so the current control efficiency of the tube current is limited, resulting in low dose modulation efficiency.

In view of this, it is necessary to provide a ray source assembly capable of improving the current control efficiency to solve the above technical problems, which will be introduced below.

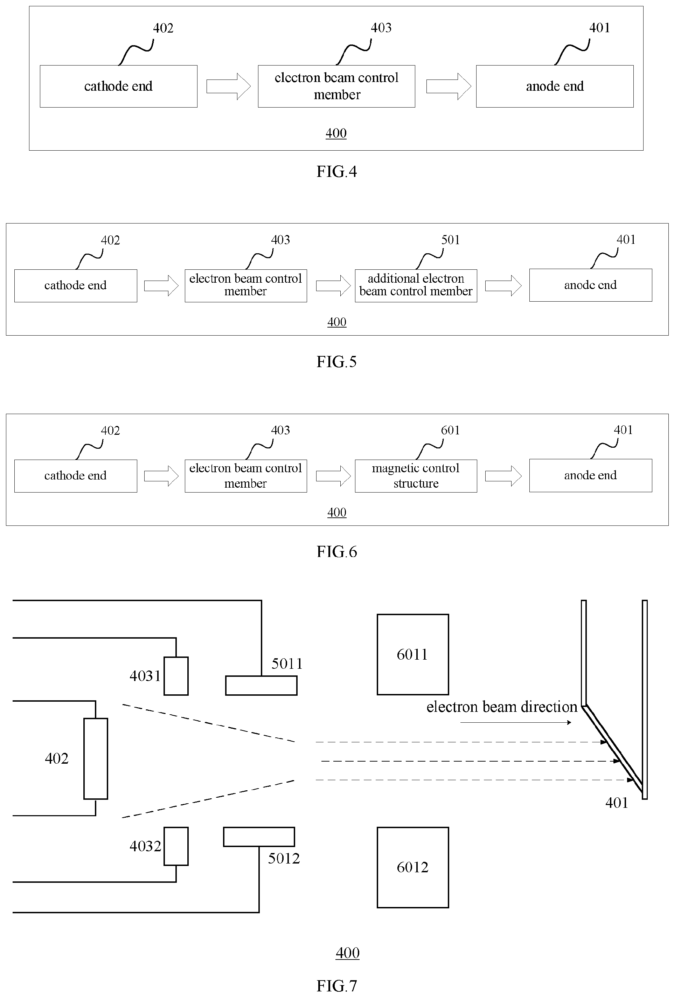

FIG. 4 is a schematic structural diagram of a ray source assembly in an exemplary embodiment. As shown in FIG. 4, the ray source assembly 400 includes an anode end 401, a cathode end 402 and an electron beam control unit 403.

The cathode end 402 is configured to emit an electron beam to the anode end 401. Exemplarily, the cathode end 402 may be connected to a negative high voltage, and the anode end 401 may be grounded or connected to a positive voltage. Optionally, the cathode end 402 may be the cathode end where the filament current is located in the CT device, and the anode end is a target area.

The anode end 401 is configured to receive the electron beam and generate rays based on the received electron beam.

During the use of the ray source assembly 400, a high-voltage electric field is applied between the cathode end 402 and the anode end 401 to make the cathode end 402 emit an electron beam to the anode end 401, so that the anode end 401 generates rays based on the received electron beam. It can be understood that the more electrons in the electron beam reaching the anode end 401, the larger the tube current.

Therefore, in order to perform dose modulation, the electron beam control unit 403 is disposed between the cathode end 402 and the anode end 401 in the present embodiment. The electron beam control unit 403 is configured to adjust the number of electrons in the electron beam that reach the anode end 401 through a control voltage. Thus, the electron beam control unit 403 can control the magnitude of the tube current. The control voltage refers to the voltage applied to the electron beam control unit 403, and the control voltage corresponding to the electron beam control unit 403 can be adjusted by means of a high-voltage generator or the like.

Exemplarily, the anode end 401 and the cathode end 402 are formed at two ends of a ray tube. The electron beam control unit 403 includes a voltage control terminal for providing a control voltage, such as an electrode structure. The voltage control terminal is disposed inside the ray tube, and a driving component for the voltage control terminal, such as a driving circuit, is disposed outside the ray tube.

As a non-limiting example, the electron beam control unit 403 can adjust the control voltage so that the number of electrons in the electron beam reaching the anode end 401 (and thus the tube current) can be changed in various ways. For example, the magnitude of the tube current (corresponding to the number of electrons reaching the anode end 401) can be adjusted as required, such as in a stepwise or smooth manner, and/or the magnitude of the tube current can be changed periodically or aperiodically (for example, randomly).

As a non-limiting example, the electron beam emitted by the cathode end 402 is set as a first electron beam, and the first electron beam has a first number of electrons. The electron beam control unit 403 can adjust the number of electrons in the first electron beam to obtain a second electron beam, and the second electron beam has a second number of electrons. The first number and the second number may be the same or different, and the specific situation may depend on the actual application requirements. The second electron beam forms tube current, and the anode end 401 may be configured to receive the second electron beam and generate rays based on the second electron beam.

Optionally, the control voltage corresponding to the electron beam control unit 403 may be less than or equal to the voltage corresponding to the cathode end 402. For example, the control voltage corresponding to the electron beam control unit 403 may be a negative voltage smaller than the voltage corresponding to the cathode end 402, such that a potential difference is formed between the cathode end 402 and the electron beam control unit 403 to block the electron beam emitted by the cathode end 402 from moving to the anode end 401, thereby reducing the number of electrons reaching the anode end 401. By adjusting the magnitude of the control voltage, the number of electrons in the electron beam that are blocked can be adjusted.

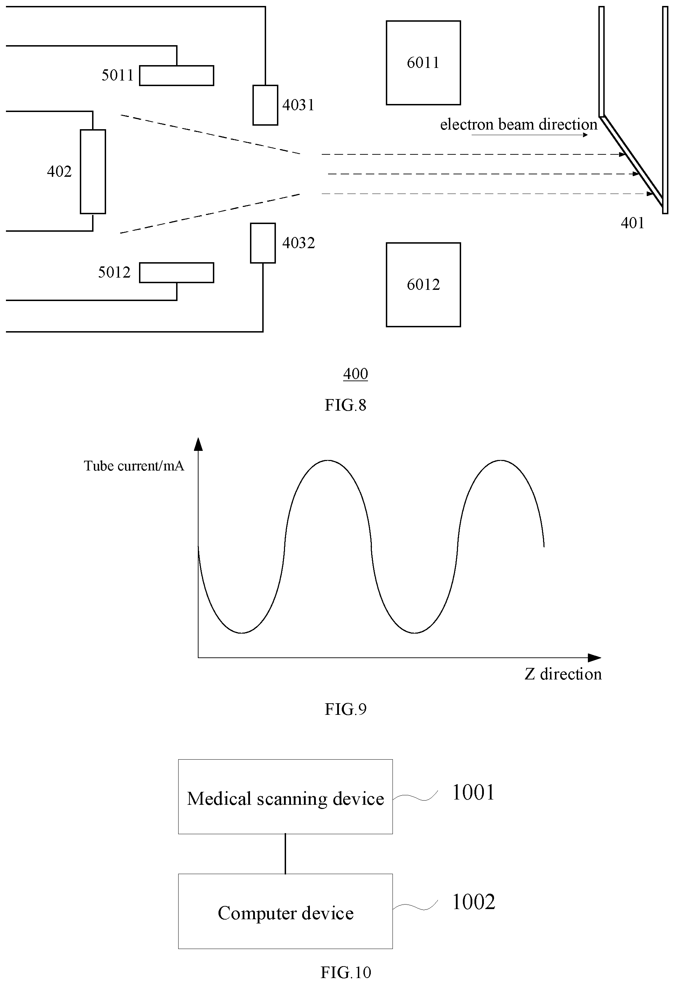

Optionally, the direction of the potential difference formed between the electron beam control unit 403 and the cathode end 402 may be parallel to the movement direction of the electron beam, or form a certain included angle with the movement direction of the electron beam. In some embodiments, the direction of the potential difference may also include both a direction parallel to the movement direction of the electron beam and a direction forming a certain included angle with the movement direction of the electron beam. For example, the direction of the potential difference is adjusted by changing the position of the voltage control terminal of the electron beam control unit 403 relative to the cathode end 402. It can be understood that the movement direction of the electron beam is the direction from the cathode end 402 to the anode end 401 (for example, the direction of the dashed arrow shown in FIGS. 7 and 8).

In the above ray source assembly 400, the cathode end 402 is configured to emit an electron beam to the anode end 401, and the anode end 401 is configured to receive the electron beam and generate rays based on the received electron beam. Since the electron beam control unit 403 can adjust the number of electrons in the electron beam that reach the anode end 401 through the control voltage, the number of electrons in the electron beam that reach the anode end 401 can be adjusted by adjusting the control voltage corresponding to the electron beam control unit 403, thereby adjusting the magnitude of the tube current corresponding to the ray source assembly 400. In this process, there is no need to change the temperature of the filament current, thus reducing the influence of the temperature change rate of the filament material and improving the current control efficiency of the tube current. Further, dose modulation can be performed quickly and efficiently through the ray source assembly 400, which can further reduce the scanning dose and improve the signal-to-noise ratio of images (for example, at different angles) generated by the ray source assembly 400.

In an exemplary embodiment, optionally, the electron beam control unit 403 includes at least two voltage control terminals, the at least two voltage control terminals form a potential difference with respect to the cathode end 402, and the direction of the potential difference is consistent with the movement direction of the electron beam.

The direction of the potential difference being consistent with the movement direction of the electron beam means that the angle difference between the direction of the potential difference and the movement direction of the electron beam is less than or equal to a preset difference, and the preset difference may be a value close to 0, for example, the preset value may be 1°, 2°, 5°, etc. Exemplarily, the direction of the potential difference may be parallel to the movement direction of the electron beam.

It can be understood that the ray source assembly 400 is usually a three-dimensional structure rather than a planar structure. That is, the electron beam emitted from the cathode end 402 is an electron beam with electrons in all three spatial directions. Therefore, in this embodiment, the electron beam control unit 403 including at least two voltage control terminals is used, and the two voltage control terminals form a potential difference with respect to the cathode end 402 respectively, so that the number of electrons in the electron beam that reach the anode end 401 can be effectively adjusted in space by using the above two potential differences. Exemplarily, the two voltage control terminals are respectively disposed on two opposite sides relative to the movement direction of the electron beam.

The difference between the control voltages corresponding to the at least two voltage control terminals is less than a preset difference. For example, the control voltages corresponding to the at least two voltage control terminals are equal.

Further optionally, the at least two voltage control terminals of the electron beam control unit 403 are symmetrically arranged relative to the movement direction of the electron beam.

As a non-limiting example, for the voltage control terminals of the electron beam control unit 403, each voltage control terminal forms a potential difference with respect to the cathode end 402. Taking the control voltage of the electron beam control unit 403 being lower than the voltage corresponding to the cathode end 402 as an example, the potential difference between the cathode end 402 and the voltage control terminal points from the cathode end 402 to the voltage control terminal.

Since the direction of the potential difference between the cathode end 402 and the voltage control terminal is consistent with the movement direction of the electron beam, the electron beam control unit can adjust the number of electrons in the electron beam that reach the anode end 401 to the maximum extent, thereby improving the current control efficiency.

In an exemplary embodiment, optionally, the magnitude of the tube current formed by the electron beam is negatively correlated with the absolute value of the control voltage.

Taking the control voltage corresponding to the electron beam control unit 403 being lower than the voltage corresponding to the cathode end 402 as an example, a potential difference is formed between the cathode end 402 and the electron beam control unit 403 to block the electron beam emitted by the cathode end 402 from moving to the anode end 401, thereby reducing the number of electrons reaching the anode end 401. Therefore, the smaller the control voltage (i.e., the larger its absolute value when negative), the greater the potential difference between the control voltage and the cathode end 402, resulting in a stronger blocking effect by the electron beam control unit 403 on the electron beam. Consequently, fewer electrons reach the anode end 401, and the tube current formed by the electron beam correspondingly decreases. Therefore, the magnitude of the tube current formed by the electron beam is negatively correlated with the absolute value of the control voltage.

In this embodiment, since the magnitude of the tube current formed by the electron beam is negatively correlated with the absolute value of the control voltage, the magnitude of the tube current formed by the electron beam can be flexibly and efficiently controlled by the absolute value of the control voltage. For example, if a larger tube current is required, the absolute value of the control voltage can be reduced.

In an exemplary embodiment, optionally, the electron beam control unit 403 is further configured to control the cross-sectional area of the electron beam and/or the position where the electron beam reaches the anode end 401 through the control voltage.

The cross-sectional area of the electron beam can affect the size of the focal point for final imaging. Exemplarily, taking electron beams with the same number of electrons as an example, assuming that the magnitude of the tube current formed thereby is 100mA (milliamperes), the size of the final formed focal point is 0.6mm*0.6mm when the cross-sectional area of the electron beam is A, and the size of the final formed focal point is 1.2mm*1.2mm when the cross-sectional area of the electron beam is B. Generally, under the same power, the smaller the size of the focal point, the better the quality of the image finally generated by the ray source assembly, that is, the quality of the image generated by the ray source assembly is negatively correlated with the cross-sectional area of the electron beam.

Optionally, the electron beam control unit 403 can control the cross-sectional area of the electron beam through the control voltages of the two voltage control terminals. For example, when the control voltage corresponding to the electron beam control unit 403 is a negative voltage, the larger the absolute value of the control voltage, the stronger the repulsion or compression effect on the electron beam, thus the smaller the cross-sectional area of the electron beam. At the same time, the control voltages of the two voltage control terminals also play a truncation role, and the number of electrons passing by the two voltage control terminals along the movement direction of the electron beam, that is, the magnitude of the tube current, can be changed by changing the magnitude of the control voltage.

Alternatively or additionally, under normal circumstances, the position where the electron beam reaches the anode end 401 after being emitted from the cathode end 402 generally does not shift, but under the control voltage, the movement direction of the electron beam between the cathode end 402 and the anode end 401 will deflect. In this way, the control voltage of the electron beam control unit 403 can control the position where the electron beam reaches the anode end 401. The position where the electron beam reaches the anode end 401 can affect the focal position of the final imaging, so the ray source assembly 400 can also be applied in some flying focal spot or spectral CT scenarios. In some embodiments, the control voltages of the two voltage control terminals are unequal, and the potential difference perpendicular to the movement direction of the electron beam generated between them determines the deflection degree of the electron beam. Therefore, the deflection of the movement direction of the electron beam can be controlled by controlling the potential difference between the control voltages of the two voltage control terminals, thereby controlling the position where the electrons reach the anode end 401.

It can be understood that in some embodiments, the control of the cross-sectional area of the electron beam and the position where it reaches the anode end 401 can be implemented at the same time. For example, the control voltage provided by the voltage control member not only provides a compression effect on the electron beam, but also controls the deflection of the electron beam.

In this embodiment, since the electron beam control unit 403 can also be used to control the cross-sectional area of the electron beam and/or the position where the electron beam reaches the anode end 401 through the control voltage, the electron beam received by the anode end 401 can be controlled more flexibly, and the application scenarios of the ray source assembly are expanded.

The above describes a way of integrating the control of the cross-sectional area of the electron beam and/or the position where the electron beam reaches the anode end 401, and the adjustment of the number of electrons reaching the anode end 401 in the electron beam control unit 403. In some embodiments, the function of controlling the cross-sectional area of the electron beam and/or the position where the electron beam reaches the anode end 401, and the function of adjusting the number of electrons reaching the anode end 401 can also be allocated to two components of the ray source assembly 400, for example, in the embodiments shown in FIGS. 5 and 6, to achieve precise control.

FIG. 5 is a schematic structural diagram of another ray source assembly in an exemplary embodiment. As shown in FIG. 5, the ray source assembly 400 further includes an additional electron beam control unit 501.

The additional electron beam control unit 501 is configured to control the cross-sectional area of the electron beam and/or the position where the electron beam reaches the anode end 401 by means of an additional control voltage. Similarly, the additional electron beam control unit 501 is also disposed between the cathode end 402 and the anode end 401. The additional control voltage refers to the voltage applied to the additional electron beam control unit 501, and the additional control voltage can be adjusted by means of a high-voltage generator or the like.

Optionally, the additional control voltage corresponding to the additional electron beam control unit 501 may be lower than the voltage corresponding to the cathode end 402. For example, the additional control voltage corresponding to the additional electron beam control unit 501 may be a negative voltage smaller than the voltage corresponding to the cathode end 402, so that the cross-sectional area and/or movement direction of the electron beam can be adjusted through the additional electron beam control unit 501, thereby controlling the cross-sectional area of the electron beam and/or the position where the electron beam reaches the anode end 401. The control principle thereof is similar to that of the above-described electron beam control unit 403.

Further optionally, the additional electron beam control unit 501 includes at least two additional voltage control terminals, such as two electrodes, and the two additional voltage control terminals can be driven by a driving circuit to generate a voltage. Similarly, the two additional voltage control terminals can be disposed inside the ray tube, and a driving component for the voltage control terminals, such as a driving circuit, is disposed outside the ray tube. The direction of the potential difference between the two additional voltage control terminals may be perpendicular to the movement direction of the electron beam, or form an angle of other degrees with the movement direction of the electron beam.

In this embodiment, since the ray source assembly 400 further includes the additional electron beam control unit 501 that controls the cross-sectional area of the electron beam and/or the position where the electron beam reaches the anode end 401, the accuracy of controlling the cross-sectional area of the electron beam and the position where the electron beam reaches the anode end 401 is improved.

In an exemplary embodiment, optionally, the cross-sectional area of the electron beam is negatively correlated with the absolute value of the additional control voltage.

In this embodiment, taking the additional control voltage corresponding to the additional electron beam control unit 501 being lower than the voltage corresponding to the cathode end 402 as an example, the additional control voltage of the additional electron beam control unit 501 will produce a repulsion or compression effect on the electron beam. Therefore, the smaller the additional control voltage (i.e., the larger its absolute value when negative), the higher the compression ability of the additional electron beam control unit 501 on the electron beam, and the smaller the cross-sectional area of the electron beam. Therefore, the cross-sectional area of the electron beam is negatively correlated with the absolute value of the additional control voltage.

In this embodiment, since the cross-sectional area of the electron beam is negatively correlated with the absolute value of the additional control voltage, the cross-sectional area of the electron beam can be flexibly and efficiently controlled by the absolute value of the additional control voltage. For example, if a larger cross-sectional area is required, the absolute value of the additional control voltage can be reduced.

In an exemplary embodiment, optionally, the additional electron beam control unit 501 includes a first additional voltage control terminal and a second additional voltage control terminal, the first additional voltage control terminal corresponds to a first additional control voltage, and the second additional voltage control terminal corresponds to a second additional control voltage.

If the absolute value of the first additional control voltage is greater than the absolute value of the second additional control voltage, the position where the electron beam reaches the anode end 401 is closer to the second additional voltage control terminal along the direction of a connecting line of the two additional voltage control terminals. If the absolute value of the first additional control voltage is less than the absolute value of the second additional control voltage, the position where the electron beam reaches the anode end 401 is closer to the first additional voltage control terminal.

That is, the additional control voltage includes a first additional control voltage and a second additional control voltage. The voltage difference between the first additional control voltage and the second additional control voltage affects the deflection direction of the electron beam. The larger the voltage difference, the larger the deflection of the electron beam, and when the voltage difference is zero, the electron beam does not deflect. Taking both the first additional control voltage and the second additional control voltage being lower than the voltage corresponding to the cathode end 402 as an example, if the first additional control voltage is lower than the second additional control voltage (i.e., the absolute value of the first additional control voltage is greater than that of the second additional control voltage when they are negative), the effect of the first additional control voltage on the electron beam is greater. Therefore, the electron beam will be closer to the second additional voltage control terminal during movement, and thus the position where the electron beam reaches the anode end 401 is closer to the second additional voltage control terminal. Similarly, when the absolute value of the first additional control voltage is less than that of the second additional control voltage, the position where the electron beam reaches the anode end 401 is closer to the first additional voltage control terminal.

In this embodiment, when the absolute value of the first additional control voltage is greater than the absolute value of the second additional control voltage, the position where the electron beam reaches the anode end 401 is closer to the second additional voltage control terminal, and when the absolute value of the first additional control voltage is less than the absolute value of the second additional control voltage, the position where the electron beam reaches the anode end 401 is closer to the first additional voltage control terminal. Therefore, the position where the electron beam reaches the anode end 401 can be flexibly controlled by the additional electron beam control unit 501.

Thus, in a specific application of the ray source assembly according to this embodiment, for example, the electron beam from the cathode end 402 can be first quickly adjusted by the electron beam control unit 403 to change the number of electrons and the cross-sectional area of the electron beam. Then, the position where the electron beam reaches the anode end 401 is changed by the additional electron beam control unit 501, and the position where the electron beam reaches the anode end 401 and the formed spot area are adjusted without changing the magnitude of the tube current. As such, precise adjustment of one or more of the cross-sectional area of the electron beam, the position where the electron beam reaches the anode end 401, and the number of electrons in the electron beam is achieved through the two-stage adjustment.

FIG. 6 is a schematic structural diagram of another ray source assembly in an exemplary embodiment. As shown in FIG. 6, the ray source assembly 400 further includes a magnetic control structure 601.

The magnetic control structure 601 is configured to control at least one of the cross-sectional area of the electron beam, the position where the electron beam reaches the anode end 401, and the number of electrons in the electron beam.

In this embodiment, since the magnetic field also has an influence on the electric field, a magnetic control structure 601 can also be disposed between the cathode end 402 and the anode end 401 in this embodiment, and the magnetic control structure 601 controls at least one of the cross-sectional area of the electron beam, the position where the electron beam reaches the anode end 401, and the number of electrons in the electron beam. In some embodiments, the magnetic control structure 601 includes a coil assembly. A magnetic field is generated by current flowing through the coil, and the direction and intensity of the magnetic field can be adjusted. The magnetic field exerts a force on the electron beam to change the movement of the electron beam. By adjusting the intensity and direction of the magnetic field, the focusing and orientation of the electron beam can be precisely controlled, thereby realizing the adjustment of at least one of the cross-sectional area of the electron beam, the position where the electron beam reaches the anode end 401, and the number of electrons in the electron beam.

In this embodiment, since the ray source assembly 400 further includes the magnetic control structure 601, and the magnetic control structure 601 is configured to control at least one of the cross-sectional area of the electron beam, the position where the electron beam reaches the anode end 401, and the number of electrons in the electron beam, the electron beam can be further controlled by using the magnetic control structure 601 to improve the flexibility of the ray source assembly. As an example, the magnetic control structure 601 may be an electromagnetic coil, an electromagnet, or various other magnetic control structures.

Thus, in a specific application of the ray source assembly according to this embodiment, for example, the electron beam from the cathode end 402 can be first quickly adjusted by the electron beam control unit 403 to change the number of electrons and the cross-sectional area of the electron beam, and then at least one of the cross-sectional area of the electron beam, the position where the electron beam reaches the anode end 401, and the number of electrons in the electron beam is adjusted by the magnetic control structure 601, so that precise adjustment of one or more of the cross-sectional area of the electron beam, the position where the electron beam reaches the anode end 401, and the number of electrons in the electron beam is achieved through two-stage adjustment.

To describe the ray source assembly in the present application more clearly, the description is given here with reference to FIGS. 7 and 8. FIGS. 7 and 8 are respectively schematic structural diagrams of another ray source assembly in embodiments of the present application. As shown in FIGS. 7 and 8, the ray source assembly 400 may include an anode end 401, a cathode end 402, and an electron beam control unit 403, an additional electron beam control unit 501, and a magnetic control structure 601, which are disposed between the anode end 401 and the cathode end 402. The electron beam control unit 403 includes a first voltage control terminal 4031 and a second voltage control terminal 4032, i.e., the two voltage control terminals described above. The additional electron beam control unit 501 includes a first additional voltage control terminal 5011 and a second additional voltage control terminal 5012. The magnetic control structure 601 includes a magnetic control terminal 6011 and a magnetic control terminal 6012. The dashed lines in FIGS. 7 and 8 are schematic diagrams of the movement trajectory of the electron beam.

Taking FIG. 7 as an example, in FIG. 7, the anode end 401 generates an electron beam and emits the electron beam to the cathode end 402. The first voltage control terminal 4031 and the second voltage control terminal 4032 can adjust the number of electrons in the electron beam that reach the anode end 401 through the control voltage. The direction of the potential difference formed between the cathode end 402 and the first voltage control terminal 4031 and the second voltage control terminal 4032 is consistent with the movement direction of the electron beam, and the magnitude of the tube current formed by the electron beam is negatively correlated with the absolute value of the control voltage.

The first additional voltage control terminal 5011 and the second additional voltage control terminal 5012 can control the cross-sectional area of the electron beam and/or the position where the electron beam reaches the anode end 401 by means of the additional control voltage. The direction of the potential difference formed between the cathode end 402 and the first additional voltage control terminal 5011 and the second additional voltage control terminal 5012 is perpendicular to the movement direction of the electron beam, and the magnitude of the tube current formed by the electron beam is negatively correlated with the absolute value of the control voltage. The cross-sectional area of the electron beam is negatively correlated with the absolute value of the additional control voltage.

If the absolute value of the additional control voltage corresponding to the first additional voltage control terminal 5011 is greater than the absolute value of the additional control voltage corresponding to the second additional voltage control terminal 5012, the position where the electron beam reaches the anode end 401 is closer to the second additional voltage control terminal 5012. If the absolute value of the additional control voltage corresponding to the first additional voltage control terminal 5011 is less than the absolute value of the additional control voltage corresponding to the second additional voltage control terminal 5012, the position where the electron beam reaches the anode end 401 is closer to the first additional voltage control terminal 5011. If the absolute value of the additional control voltage corresponding to the first additional voltage control terminal 5011 is equal to the absolute value of the additional control voltage corresponding to the second additional voltage control terminal 5012, the electron beam does not deflect.

The magnetic control terminal 6011 and the magnetic control terminal 6012 can further control at least one of the cross-sectional area of the electron beam, the position where the electron beam reaches the anode end 401, and the number of electrons in the electron beam.

FIG. 8 shows that, on the basis of FIG. 7, the relative positions of the electron beam control unit 403 and the additional electron beam control unit 501 are exchanged, and the other principles are the same, which will not be repeated here. In FIG. 7, the distance between the electron beam control unit 403 and the cathode end 402 is less than the distance between the additional electron beam control unit 501 and the cathode end 402, that is, along the movement direction of the electron beam, the electron beam control unit 403 is located upstream of the additional electron beam control unit 501. The voltage of the additional electron beam control unit 501 may be higher than the voltage of the electron beam control unit 403, that is, when both are negative voltages, the absolute value of the voltage of the electron beam control unit 403 is greater than that of the additional electron beam control unit 501. Therefore, the magnitude of the tube current can be first adjusted by the control voltage of the electron beam control unit 403, and then the cross-sectional area and deflection of the electron beam can be adjusted by the additional electron beam control unit 501. The magnitude of the tube current is not affected during the adjustment of the cross-sectional area and deflection of the electron beam. In FIG. 8, the distance between the electron beam control unit 403 and the cathode end 402 is greater than the distance between the additional electron beam control unit 501 and the cathode end 402, which can reduce the engineering implementation difficulty of the ray source assembly 400 and is conducive to actual production.

FIG. 9 is a schematic diagram of a dose modulation in an embodiment of the present application. FIG. 9 shows an actual change curve of the tube current during the modulation process by using the ray source assembly 400 provided in this embodiment. As shown in FIG. 9, by using the method provided in this embodiment, the upper and lower limits of the actual tube current can be increased, thereby improving the current control efficiency of the tube current and thus improving the efficiency of dose modulation.

As an example, during dose modulation, the filament current (corresponding to the current passing through the cathode end 402) can be maintained at a reasonable and relatively large current value in advance. According to the planned dose modulation curve, during the scanning with rays, each time scanning data is sampled, the change of the control voltage can be regulated by the electron beam control unit 403 (e.g., a high-voltage generator) according to the required tube current corresponding to the expected ray dose, so as to realize the control of the tube current according to the sampling frequency, thereby realizing a fast modulation capability relative to the conventional dose modulation, for example, as shown in FIG. 9.

In an embodiment, a ray control method applicable to the above ray source assembly is also provided. FIG. 10 is an application environment diagram of a ray control method in an embodiment of the present application. A medical scanning device 1001 communicates with the computer device 1002 in a wired or wireless manner. The medical scanning device 1001 includes, but is not limited to, a CT (Computed Tomography) device, a PET-CT device, an X-ray device, or the like. It can be understood that the medical scanning device 1001 should include a ray source, and the ray source may include the above-mentioned ray source assembly 400.

The computer device 1002 may be, but is not limited to, various personal computers, notebook computers, smart phones, tablet computers and portable wearable devices, and the portable wearable devices may be smart watches, smart bracelets, head-mounted devices, etc. Certainly, the computer device 1002 may also be implemented by an independent server or a server cluster composed of multiple servers.

In some embodiments, the computer device 1002 may also be disposed inside the medical scanning device 1001, and the computer device 1002 includes, but is not limited to, a Central Processing Unit (CPU), and may also include at least one of a Digital Signal Processor (DSP), a Field-Programmable Gate Array (FPGA), or other programmable logic devices.

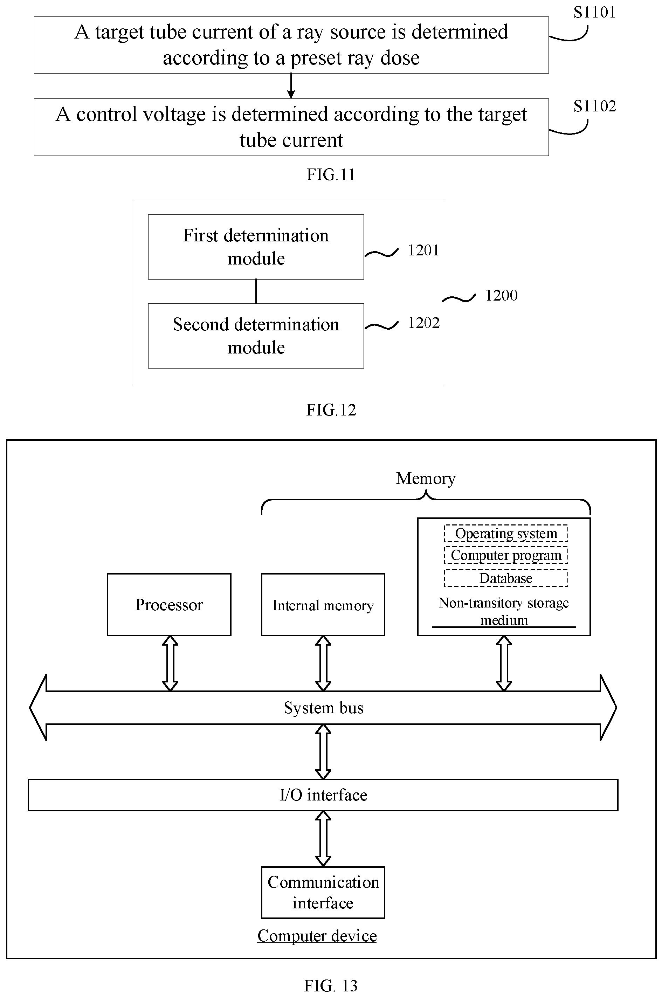

FIG. 11 is a schematic flow chart of a ray control method in an exemplary embodiment. As shown in FIG. 11, a ray control method is provided, and the method may include steps S1101 to S1102. As a non-limiting example, a part or all of the method may be executed by the computer device in FIG. 10.

In step S1101, a target tube current of a ray source is determined according to a preset ray dose.

In this embodiment, the preset ray dose is used to indicate the scanning dose required during the scanning process. Optionally, the preset ray dose may be a time-related sequence. For example, time point 1 to time point 5 correspond to preset ray dose 1, time point 6 to time point 10 correspond to preset ray dose 2, and so on.

The preset ray dose may be a parameter pre-planned and stored in the computer device, a parameter determined by the computer device in response to a user's input operation, or a parameter determined by the computer device after analyzing information such as the height, weight and gender of the scanned object, which is not limited in this embodiment.

It can be understood that the tube currents of the ray source corresponding to different ray doses are different. A first correspondence relationship between different ray doses and different tube currents of the ray source can be obtained, and then a target tube current of the ray source is determined according to the preset ray dose and the first correspondence relationship. Exemplarily, in the first correspondence relationship, a first ray dose corresponds to a first tube current, and a second ray dose corresponds to a second tube current. In the preset ray dose, a first preset ray dose corresponds to time point 1 to time point 5, and a second preset ray dose corresponds to time point 6 to time point 10. Then the target tube current of the ray source from time point 1 to time point 5 is the first tube current, and the target tube current of the ray source from time point 6 to time point 10 is the second tube current.

In step S1102, the control voltage is determined according to the target tube current.

In this embodiment, after the target tube current is determined, the control voltage can be determined according to the target tube current, so as to adjust the number of electrons in the electron beam emitted from the cathode end of the ray source that reach the anode end of the ray source through the control voltage, thereby adjusting the tube current corresponding to the ray source to the target tube current. That is, the control voltage is configured to adjust the number of electrons in the electron beam emitted from the cathode end of the ray source that reach the anode end of the ray source. For the principle that the control voltage adjusts the number of electrons reaching the anode end of the ray source, reference may be made to the above embodiments, which will not be repeated here.

Optionally, a second correspondence relationship between different control voltages and different tube currents can be obtained, and the second correspondence relationship may be, but is not limited to, in a form of a two-dimensional table or a mathematical model. The control voltage can then be determined according to the target tube current and the second correspondence relationship. Exemplarily, in the second correspondence relationship, a first tube current corresponds to a first control voltage, and a second tube current corresponds to a second control voltage. Then it can be determined that the control voltage from time point 1 to time point 5 is the first control voltage, and the control voltage from time point 6 to time point 10 is the second control voltage.

In some embodiments, the ray control method can also be applied to some clinical applications of dose modulation, such as ECG (electrocardiogram)-DOM in cardiac scanning, to reduce the dose and modulation efficiency of cardiac scanning. It can be understood that the ray control method can also be applied to image examinations of regions such as the head and lungs.

In the above ray control method, the target tube current of the ray source is determined according to the preset ray dose, and the control voltage is determined according to the target tube current. Since the control voltage is configured to adjust the number of electrons in the electron beam emitted from the cathode end of the ray source that reach the anode end of the ray source, the number of electrons in the electron beam that reach the anode end can be adjusted by adjusting the control voltage, thereby adjusting the magnitude of the tube current to adjust the scanning dose. In this process, there is no need to change the temperature of the filament current, thus reducing the influence of the temperature change rate of the filament material and improving the efficiency of dose modulation.

In an exemplary embodiment, the way of adjusting the tube current in the prior art can also be combined with the way of adjusting the tube current by the control voltage in this embodiment. For example, the dose modulation method in this embodiment is used in the X direction, and the conventional dose modulation method in the prior art is used in the Z direction. This approach can take into account the dose adjustment in both the X direction and the Z direction, and the average current of the filament in the ray source can be reduced, avoiding the filament current being at a high value continuously, and prolonging the service life of the filament and improving the reliability and service life of the tube.

In an exemplary embodiment, a target focal point requirement can also be obtained, and an additional control voltage is determined according to the target focal point requirement. The additional control voltage is configured to adjust the cross-sectional area of the electron beam emitted from the cathode end of the ray source and/or the position where the electron beam reaches the anode end of the ray source. The target focal point requirement includes a focal point size requirement and/or a focal point position requirement.

It can be understood that if the target focal point requirement includes a focal point size requirement, the additional control voltage can adjust the cross-sectional area of the electron beam emitted from the cathode end of the ray source, and if the target focal point requirement includes a focal point position requirement, the additional control voltage can adjust the position where the electron beam reaches the anode end of the ray source.

Optionally, a third correspondence relationship between different target focal point requirements and additional control voltages can be obtained, and after the target focal point requirement is obtained, the additional control voltage is determined according to the target focal point requirement and the third correspondence relationship.

For the principle of adjusting the cross-sectional area of the electron beam emitted from the cathode end of the ray source and the position where the electron beam reaches the anode end of the ray source, reference may be made to the above embodiments, which will not be repeated here.

In an exemplary embodiment, the cathode interface connected to the cathode end 402 may support fast tube current (mA) modulation. Optionally, fast mA modulation may also be combined with the switching of the kilovoltage peak (kV) applied between the cathode end 402 and the anode end 401, where a high kV can correspond to a low mA and a low kV corresponds to a high mA to achieve dose matching, so as to realize dose balance in spectral scanning.

In spectral scanning, various high-voltage electric fields are applied between the cathode end and the anode end of the ray source. The energy of the electron beams are different if the magnitudes of the high-voltage electric field are different. Therefore, under the same tube current, the scanning doses corresponding to different high-voltage electric fields are also different. In order to make the scanning doses corresponding to different high-voltage electric fields the same, the magnitude of the tube current can be reduced by the control voltage when the high-voltage electric field is high, and the magnitude of the tube current can be increased when the high-voltage electric field is low.

In an exemplary embodiment, the fast dose modulation function and fast kV switching can be combined to realize the dose modulation function during the fast kV switching process. For example, in an actual implementation example, the dose modulation curves can be planned according to the scanning parameters of different kilovolts respectively, and then two or more dose modulation curves are combined and controlled, so as to realize the dose modulation function during the kV switching process.

In other words, multiple preset voltages can be obtained, and a control voltage corresponding to each preset voltage is determined according to the preset voltage, so as to adjust the number of electrons in the electron beam emitted from the cathode end of the ray source that reach the anode end of the ray source through the control voltage, thereby adjusting the tube current corresponding to the ray source to the required tube current. The preset voltage is used to indicate multiple groups of high-voltage electric fields in fast kV.

Further optionally, a dose modulation curve corresponding to each preset voltage can be obtained, and then a control voltage corresponding to each preset voltage is determined according to the preset voltage and the dose modulation curve corresponding to each preset voltage. The dose modulation curve corresponding to the preset voltage includes a correspondence relationship between different scanning doses and control voltages. Exemplarily, in a first dose modulation curve corresponding to a first preset voltage, a first scanning dose corresponds to a first control voltage, and in a first dose modulation curve corresponding to a second preset voltage, the same first scanning dose corresponds to a second control voltage. Therefore, in order to make the scanning dose corresponding to the first preset voltage the same as the scanning dose corresponding to the second preset voltage, the first control voltage is used under the first preset voltage, and the second control voltage is used under the second preset voltage.

It should be understood that although the various steps in the flow charts involved in the above embodiments are displayed in sequence according to the arrow indications, these steps are not necessarily executed in sequence according to the arrow indications. Unless explicitly stated herein, the execution of these steps has no strict order limit, and these steps can be executed in other orders. Moreover, at least some of the steps in the flow charts involved in the above embodiments may include multiple steps or multiple stages, and these steps or stages are not necessarily executed at the same time, but can be executed at different times, and the execution order of these steps or stages is not necessarily sequential, but can be performed alternately or alternately with at least a part of other steps or steps in other steps.

Based on the same inventive concept, the embodiments of the present application also provide a ray control apparatus for performing the above-mentioned ray control method. The implementation solution for solving the problem provided by the device is similar to the implementation solution recorded in the above method, so the specific limitations in one or more ray control apparatus embodiments provided below can be referred to the limitations on the ray control method above, and will not be repeated here.

FIG. 12 is a structural block diagram of a ray control and adjustment apparatus in embodiments of the present application. As shown in FIG. 12, a ray control apparatus is provided, including a first determination module 1201 and a second determination module 1202.

The first determination module 1201 is configured to determine a target tube current of a ray source according to a preset ray dose.

The second determination module 1202 is configured to determine a control voltage according to the target tube current. The control voltage is configured to adjust the number of electrons in the electron beam emitted from a cathode end of the ray source that reach an anode end of the ray source.

In the above ray control apparatus, the target tube current of the ray source is determined according to the preset ray dose, and the control voltage is determined according to the target tube current. Since the control voltage is configured to adjust the number of electrons in the electron beam emitted from the cathode end of the ray source that reach the anode end of the ray source, the number of electrons in the electron beam that reach the anode end can be adjusted by adjusting the control voltage, thereby adjusting the magnitude of the tube current to adjust the scanning dose. In this process, there is no need to change the temperature of the filament current, thus reducing the influence of the temperature change rate of the filament material and improving the efficiency of dose modulation.

Each module in the above ray control apparatus can be implemented in whole or in part by software, hardware and a combination thereof. The above modules can be embedded in or independent of the processor in the computer device in the form of hardware, or stored in the memory in the computer device in the form of software, so that the processor can call and execute the operations corresponding to the above modules.

FIG. 13 is an internal structural diagram of a computer device in embodiments of the present application. In an exemplary embodiment, a computer device is provided, which may be a server, and its internal structural diagram may be as shown in FIG. 13. The computer device includes a processor, a memory, an Input/Output (I/O) interface and a communication interface. The processor, the memory and the input/output interface are connected through a system bus, and the communication interface is connected to the system bus through the input/output interface. The processor of the computer device is used to provide computing and control capabilities. The memory of the computer device includes a non-transitory storage medium and an internal memory. The non-transitory storage medium stores an operating system, a computer program and a database. The internal memory provides an environment for the operation of the operating system and the computer program in the non-transitory storage medium. The database of the computer device is used to store relevant data. The input/output interface of the computer device is used to exchange information between the processor and external devices. The communication interface of the computer device is used to communicate with an external terminal through a network connection. The computer program, when executed by the processor, a ray control method is performed.

Those skilled in the art can understand that the structure shown in FIG. 13 is merely a block diagram of a part of the structure related to the solution of the present application, and does not constitute a limitation on the computer device to which the solution of the present application is applied. A specific computer device may include more or fewer components than those shown in the figure, or combine some components, or have a different component arrangement.

In an exemplary embodiment, a computer device is provided, including a memory and a processor. The memory has a computer program stored therein. The processor, when executing the computer program, implements the following steps:
determining a target tube current of a ray source according to a preset ray dose; and
determining a control voltage according to the target tube current, where the control voltage is configured to adjust the number of electrons in the electron beam emitted from a cathode end of the ray source that reach an anode end of the ray source.

In an embodiment, a computer-readable storage medium is provided, having a computer program stored therein. The computer program, when executed by a processor, implements the following steps:
determining a target tube current of a ray source according to a preset ray dose; and
determining a control voltage according to the target tube current, where the control voltage is configured to adjust the number of electrons in the electron beam emitted from a cathode end of the ray source that reach an anode end of the ray source.

In an embodiment, a computer program product is provided, including a computer program. The computer program, when executed by a processor, implements the following steps:
determining a target tube current of a ray source according to a preset ray dose; and
determining a control voltage according to the target tube current, where the control voltage is configured to adjust the number of electrons in the electron beam emitted from a cathode end of the ray source that reach an anode end of the ray source.

Those of ordinary skill in the art can understand that all or part of the processes in the methods of the above embodiments can be completed by instructing relevant hardware through a computer program. The computer program can be stored in a non-transitory computer-readable storage medium, and when executed, the computer program can include the processes of the embodiments of the methods described above. Any reference to a memory, database or other medium used in the embodiments provided herein may include at least one of non-transitory and volatile memories. Non-transitory memories may include read-only memory (ROM), magnetic tape, floppy disk, flash memory, optical memory, high-density embedded non-transitory memory, resistive random access memory (ReRAM), magnetoresistive random access memory (MRAM), ferroelectric random access memory (FRAM), phase change memory (PCM), graphene memory, etc. Volatile memories may include random access memory (RAM) or external cache memory, etc. By way of illustration and not limitation, RAM may take many forms, such as static random access memory (SRAM) or dynamic random access memory (DRAM), etc. The databases involved in the embodiments provided herein may include at least one of relational databases and non-relational databases. Non-relational databases may include blockchain-based distributed databases, but are not limited thereto. The processors involved in the embodiments provided herein may be general-purpose processors, central processing units, graphics processors, digital signal processors, programmable logic devices, quantum computing-based data processing logic devices, etc., but are not limited thereto.

The technical features of the above embodiments can be combined arbitrarily. For the sake of brevity of description, not all possible combinations of the technical features in the above embodiments are described. However, as long as there is no contradiction in the combination of these technical features, they shall be deemed to be within the scope recorded in this specification.

The above-described embodiments merely represent several implementation manners of the present application, and the descriptions thereof are relatively specific and detailed, but shall not be construed as limiting the patent scope of the present application. It should be noted that, for those of ordinary skill in the art, several variations and improvements can be made without departing from the concept of the present application, and these all fall within the protection scope of the present application. Therefore, the protection scope of the present application shall be subject to the appended claims.

## Claims

1. A ray source assembly, comprising:
an anode end (401) configured to receive an electron beam and generate rays based on the received electron beam;
a cathode end (402) configured to emit the electron beam to the anode end (401); and
an electron beam control unit (403) disposed between the cathode end (402) and the anode end (401) and configured to adjust the number of electrons in the electron beam that reach the anode end (401) through a control voltage.

2. The ray source assembly according to claim 1, wherein the electron beam control unit (403) comprises at least two voltage control terminals, the at least two voltage control terminals form a potential difference with respect to the cathode end (402), and a direction of the potential difference is consistent with a movement direction of the electron beam.

3. The ray source assembly according to claim 1 or 2, wherein the electron beam control unit (403) is configured such that a magnitude of a tube current formed by the electron beam is negatively correlated with a potential difference between the cathode end (402) and the electron beam control unit (403).

4. The ray source assembly according to any one of claims 1 to 3, wherein the control voltage is a negative voltage and lower than a voltage of the cathode end (402), and the electron beam control unit (403) is configured such that a magnitude of a tube current formed by the electron beam is negatively correlated with an absolute value of the control voltage.

5. The ray source assembly according to any one of claims 1 to 4, wherein the electron beam control unit (403) is further configured to control a cross-sectional area of the electron beam and/or a position where the electron beam reaches the anode end (401) through the control voltage.

6. The ray source assembly according to any one of claims 1 to 5, wherein the electron beam control unit (403) comprises a first voltage control terminal (4031) and a second voltage control terminal (4032) respectively located on two opposite sides relative to a movement direction of the electron beam.

7. The ray source assembly according to claim 6, wherein the first voltage control terminal (4031) and the second voltage control terminal (4032) are configured to control, based on corresponding control voltages, the number of electrons passing by the first voltage control terminal (4031) and the second voltage control terminal (4032) along the movement direction of the electron beam.

8. The ray source assembly according to claim 6 or 7, wherein the first voltage control terminal (4031) and the second voltage control terminal (4032) are configured to control deflection of the electron beam based on corresponding control voltages.

9. The ray source assembly according to any one of claims 1 to 8, wherein the ray source assembly further comprises:
an additional electron beam control unit (501) configured to control a cross-sectional area of the electron beam and/or a position where the electron beam reaches the anode end (401) through an additional control voltage.

10. The ray source assembly according to claim 9, wherein the additional control voltage is a negative voltage and lower than a voltage of the cathode end (402), and the additional electron beam control unit (501) is configured such that the cross-sectional area of the electron beam is negatively correlated with an absolute value of the additional control voltage.

11. The ray source assembly according to claim 9 or 10, wherein the additional electron beam control unit (501) comprises a first additional voltage control terminal and a second additional voltage control terminal, the first additional voltage control terminal provides a first additional control voltage, the second additional voltage control terminal provides a second additional control voltage, and the additional electron beam control unit (501) is configured to control deflection of the electron beam based on the first additional control voltage and the second additional control voltage.

12. The ray source assembly according to any one of claims 9 to 11, wherein, along the movement direction of the electron beam, the electron beam control unit (403) is located upstream of the additional electron beam control unit (501).

13. The ray source assembly according to any one of claims 1 to 12, wherein the ray source assembly further comprises:
a magnetic control structure (601) configured to control at least one of a cross-sectional area of the electron beam, a position where the electron beam reaches the anode end (401), and the number of electrons in the electron beam.

14. A ray control method, applied to the ray source assembly according to any one of claims 1 to 13, comprising:
determining a target tube current of a ray source according to a preset ray dose; and
determining the control voltage according to the target tube current.

15. A computer-readable storage medium having a computer program stored therein, wherein the computer program, when executed by a processor, implements steps of the ray control method according to claim 14.
